# EUROPEAN PATENT APPLICATION

(11) **EP 2 633 826 A2**
(43) Date of publication of application: **04.09.2013**
(21) Application number: 12185523.3
(22) Date of filing: 21.09.2012
(51) Int. Cl.: A61B 17/34

(54) **Implanting apparatus**

(30) Priority: 29.02.2012 KR 20120020693
(71) Applicant: Ohsung Electronics Co., Ltd., Gyeongsangbuk-do 730-902 (KR)
(72) Inventor: Kim, Jung Chul, 706-766 Daegu (KR); Kim, Moon Kyu, 706-931 Daegu (KR); Choi, Un Ha, 702-050 Daegu (KR); Kang, Bo Sung, 730-807 Gumi-si (KR); Shin, Jeong Hun, 706-170 Daegu (KR)
(74) Representative: Epping - Hermann - Fischer

(57) **Abstract**

Provided is an implanting apparatus. The implanting apparatus includes: a casing; an implanting needle portion in the casing; a mandrel portion forwardly and backwardly movable in relation to the implanting needle portion; an elastic member disposed between the implanting needle portion and the mandrel portion; a movable portion movable forwards and backwards to move the implanting needle portion; a spring fixed relative to the casing so as to fix a position of the mandrel portion when the implanting needle portion is moved; and a transforming portion movable together with the implanting needle portion for transforming the spring. Therefore, musculoskeletal ailments of medical personnel arising from an excessive use of an hair implanting device may be prevented, and hair implantation may be performed accurately, regularly, swiftly, and without depending on a skill of medical personnel.

## Description

This application claims priority to Korean Patent Application No. 10-2012-0020693, filed February 29, 2012, which is hereby incorporated by reference.

### BACKGROUND

The present disclosure relates to an implanting apparatus for implanting hair.

In general, a hair implantation device is used to implant new hair on a scalp to replace hair damaged by diseases or accidents.

Single hair graft, which is one of the most widespread hair implantation techniques using such hair implantation device, causes no bleeding or scar since hair roots are separated one by one and implanted by a hair implanting needle. Also, the direction, angle and density of the hair roots may be adjusted as desired to maintain a natural shape of the hair after an implantation surgery. During such implantation surgery based on single hair graft, the implanting device places the hair root, which is the root connected to the hair, at a front part of the hair implanting needle in the implanting device and then the hair implanting needle is inserted into the scalp so that the hair root is accommodated by the scalp along the needle while the needle forms a hole. Here, a movable mandrel which moves in the implanting needle is used in most cases to put the hair root into a skin tissue in the scalp.

The following tasks may be necessary for the operation of the implanting needle and the mandrel. Firstly, the main body of the implanting device must be pressed toward the scalp to thrust the implanting needle into the scalp. Secondly, the handle must be pressed to take the implanting needle out of the scalp with the mandrel supporting the hair root.

The tasks mentioned above cause great inconvenience during medical treatment. Also, the implanting device causes inconvenience since thousands of hair roots must be implanted at a time. Such inconvenience is increasingly led to musculoskeletal ailments of medical personnel.

### SUMMARY

Embodiments provide an implanting apparatus which may be used conveniently, prevent musculoskeletal ailments of medical personnel arising from an excessive use of an hair implanting device, and increase the speed and accuracy of hair implantation to suit needs of a patient.

In one embodiment, an implanting apparatus includes: a casing; an implanting needle portion in the casing; a mandrel portion forwardly and backwardly movable in relation to the implanting needle portion; an elastic member disposed between the implanting needle portion and the mandrel portion; a movable portion movable forwards and backwards to move the implanting needle portion; a spring fixed relative to the casing so as to fix a position of the mandrel portion when the implanting needle portion is moved; and a transforming portion movable together with the implanting needle portion for transforming the spring. Since medical personnel have only to move the movable portion automatically, hair implantation may be made automatic and convenient.

The casing may be separated into multiple pieces, and a concave portion may be disposed at a front end of a front casing disposed at a front to guide a direction of hair root insertion. Therefore, medical personnel may observe a direction of hair implantation with convenience and insert a hair root with accuracy. The implanting apparatus may further include a motor portion to move the movable portion forwards and backwards so that medical personnel have only to hold the implanting apparatus with a certain weight for hair implantation. At least one of a rotary movement between the casing and the implanting needle portion, a rotary movement between the movable portion and the casing, and a rotary movement between the implanting needle portion and the mandrel portion may be restricted so that component interaction may be stable.

In another embodiment, an implanting apparatus includes: an operation start signal generator which generates an operation start signal when an outside signal is input; a control unit which puts a motor into operation by receiving the signal generated by the operation start signal generator; and the motor which moves the implanting needle portion forwards and backwards by following the control unit. According to the embodiment, the implanting apparatus is automated for the convenience of medical personnel. The implanting apparatus may further include an operation unit for controlling an operation status of the motor. Therefore, the implanting apparatus may be suitable for different implantation needs.

According to the embodiments, safety and accurate hair implantation may be secured for medical personnel, and hair implantation may be made swift and convenient. Also, hair implantation costs may be reduced, and patient satisfaction may be increased.

The details of one or more embodiments are set forth in the accompanying drawings and the description below. Other features will be apparent from the description and drawings, and from the claims.

### BRIEF DESCRIPTION OF THE DRAWINGS

Fig. 1 is a sectional view illustrating an implanting apparatus according to an embodiment.

Fig. 2 is a side view illustrating a front casing of the implanting apparatus.

Fig. 3 is a view illustrating a state in which the front casing and a middle casing are separated from each other.

Fig. 4 is a sectional view taken along line I-I' of FIG. 1.

Fig. 5 is a view illustrating a state in which the front casing, an implanting needle portion, and a mandrel portion are separated from one another.

Fig. 6 is a control block diagram illustrating the implanting apparatus.

Fig. 7 is a time-series diagram illustrating mechanical operations of the implanting apparatus.

### DETAILED DESCRIPTION OF THE EMBODIMENTS

Reference will now be made in detail to the embodiments of the present disclosure, examples of which are illustrated in the accompanying drawings. The invention may, however, be embodied in many different forms and should not be construed as being limited to the embodiments set forth herein; rather, that alternate embodiments included in other retrogressive inventions or falling within the spirit and scope of the present disclosure can easily be derived through adding, altering, and changing, and will fully convey the concept of the invention to those skilled in the art.

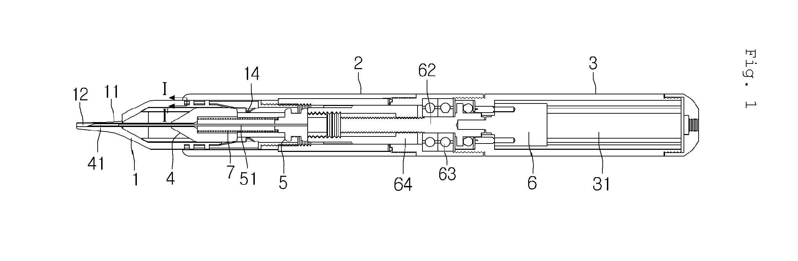

Fig. 1 is a sectional view illustrating an implanting apparatus according to an embodiment.

Referring to Fig. 1, the implanting apparatus includes an implanting needle portion 4 which includes an implanting needle 41 penetrating a scalp, a mandrel portion 5 which is superimposed in part with the implanting needle portion to support a hair root in the scalp, a movable portion 64 which moves the implanting needle portion 4 and the mandrel portion 5 forwards and backwards, and a portion which moves the movable portion 64. The implanting apparatus further includes a casing where the implanting needle portion 4, the mandrel portion 5, and the movable portion 64 are accommodated.

The casing includes a front casing 1 where the implanting needle portion 4 and the mandrel portion 5 are placed, a rear casing 3 where a power supply portion 31 and a motor portion 6 are placed, and a middle casing 2 where the movable portion 64 and a bearing portion 63 are placed.

Operation of the implanting apparatus is described below.

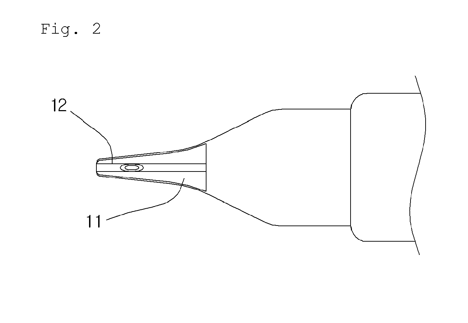

First of all, the hair root is placed at an inclined opening at the front of the implanting needle 41 of the implanting needle portion 4. The implanting needle 41 is disposed at a pin hole 12 penetrating a concave portion 11 of the front end of the front casing 1. Therefore, the implanting needle 41 is not exposed at all while not in operation and an erroneous stabbing by the sharp front end of the implanting needle 41 may be prevented for higher safety. The shapes of the pin hole 12 and the concave portion 11 are described in Fig. 2.

When an operation start signal from outside starts the operation after the hair root is placed, the motor portion 6 is put into operation by the power supplied by the power supply portion 31, and a driving shaft 62 rotates while being supported by the bearing portion 63. The rotary movement of the driving shaft 62 is converted into the rectilinear movement of the movable portion 64. For the conversion of the direction of the rotary / rectilinear movements, the driving shaft 62 and the movable portion 64 are screw connected with each other, and the movable portion 64 may be restricted so that only a linear directional movement may be possible in relation to the middle casing 2.

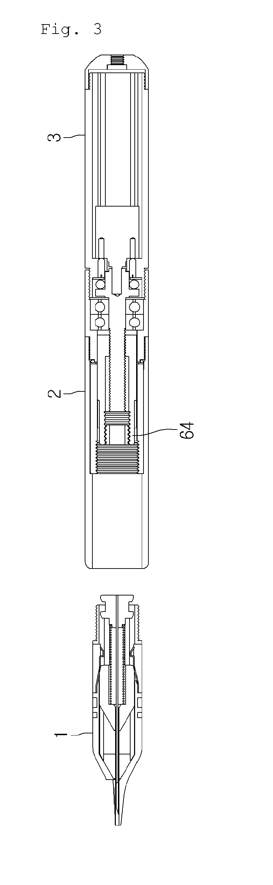

The movements mentioned above may be more clearly understood by referring to Fig. 3. The restriction of the movement is described later.

The front end of the movable portion 64 is supported by a rear part of the implanting needle portion 4. Therefore, the implanting needle portion 4 moves forwards or backwards with the movable portion 64 when the movable portion 64 moves forwards or backwards.

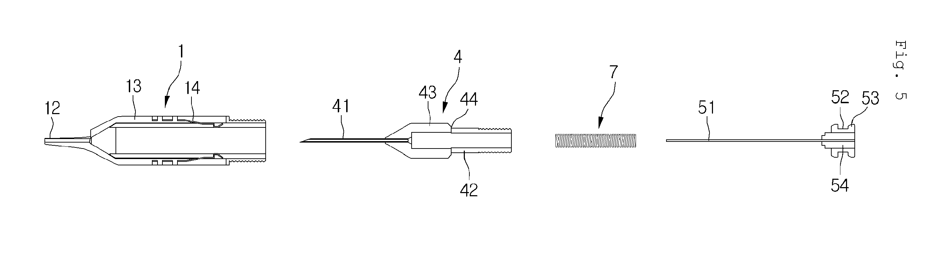

Fig. 5 is a view illustrating a state in which the front casing 1, the implanting needle portion 4, and the mandrel portion 5 are separated from one another.

Referring to Fig. 5, the hair root may be supported in the scalp when the implanting needle portion 4 is taken out of the scalp since a mandrel 51 of the mandrel portion 5 is inserted into the implanting needle 41. The mandrel 51 is supported by a mandrel supporting portion 54. Specifically, the mandrel supporting portion 54 may be moved along a slit extending in a lengthwise direction of an implanting needle supporting portion 42. Therefore, the mandrel supporting portion 54 may not rotate relatively in relation to implanting needle supporting portion 42. Also, a pair of holding portions 53 that may be separated in frontward and backward directions are disposed at one of opposing outer circumferences of the mandrel supporting portion 54, and a groove 52 is disposed in a middle of the holding portion 53. The groove 52 is where a plate spring 14 supported by the front casing 1 is inserted. When the plate spring 14 is held by the holding portion 53, the mandrel supporting portion 54 does not move in relation to the front casing 1.

The implanting needle portion 4 includes the implanting needle 41 and the implanting needle supporting portion 42. The implanting needle supporting portion 42 includes a guide structure so that the implanting needle 41 may not rotate in relation to the front casing 1 but move forwards or backwards. Therefore, the direction in which the hair root is inserted in relation to the scalp determined by the concave portion 11 may be maintained without change when the implanting needle 41 is taken out. Therefore, the direction of hair root accommodation guided by the implanting apparatus and the direction of hair growth induced therefrom may be maintained as desired.

To restrict rotation, a groove portion 13 is disposed in the front casing 1, and a protruding portion 43 is disposed at an outer surface of the implanting needle supporting portion 42. Also, a rear part of the protruding portion 43 is inclined in order to extend the plate spring 14 and release the plate spring 14 from the mandrel supporting portion 54, the operation of which is detailed later. Since the groove portion 13 and the protruding portion 43 are guided only forwards and backwards through a male-female operation, the implanting needle portion 4 may move only forwards and backwards without rotating. The operation mentioned above may be realized in the same manner by the movable portion 64 and the middle casing 2.

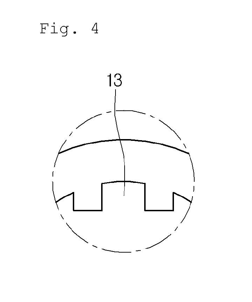

Fig. 4 is a sectional view taken along line I-I' of Fig. 1.

Referring to Fig. 4, the groove portion 13 is disposed at a part of the front casing 1, and the protruding portion 43, which may be moved forwards and backwards, is disposed in the groove portion 13.

The structure may be realized in the same manner by the movable portion 64 and the middle casing 2. For example, a protrusion may be disposed at the movable portion 64, and a concavity may be disposed at the middle casing 2. In this manner, the movable portion 64 may move only forwards and backwards without rotating in relation to the middle casing 2.

The description above provides full understanding for the internal structure and overall operation of the implanting apparatus. In the meantime, medical personnel are required to perform the following tasks. With the implanting apparatus held, the motor portion 6 of the implanting apparatus is put into operation while the front end of the front casing 1 remains in contact with the scalp of a patient. Here, different methods may be used to order a relative movement between putting the implanting apparatus into operation with the front end of the front casing 1 coming into contact with the scalp and putting the motor portion 6 into operation.

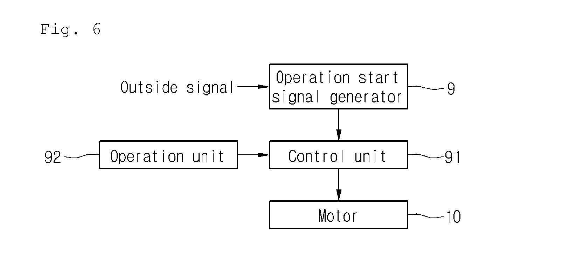

The operation mentioned above may be more fully understood by referring to Fig. 6. A signal generated by an outside signal at an operation start signal generator 9 is transmitted to a control unit 91, and the control unit 91 puts a motor 10 into operation to start the implanting apparatus. Here, the motor 10 may be on par with the motor portion 6.

Examples of the outside signal include: a signal generated by a certain button disposed at an outer surface of the casing 1, the button being pressed by medical personnel when the operation is ready; a signal generated by a pressure sensor disposed at an end of the front casing 1, the pressure sensor and the scalp being pressed by each other when the operation is ready; and a signal that is converted, because of the scalp, from light of an optical sensor disposed at a certain part of the front casing 1 when the operation is ready.

When the outside signal is transmitted to the operation start signal generator 9, the operation start signal generator 9 transmits the signal to the control unit 91 in the form of an electric signal. The control unit 91 controls the motor 10 by generating an output signal so that the implanting device is put into operation. Details of the operation of the implanting device may be fully understood by the description above and Fig. 1 through Fig. 5.

The implanting apparatus may further include an operation unit 92. The operation unit 92 may function as an interface with which medical personnel adjust a driving speed or distance of the motor depending on the status of the patient. Therefore, treatment options suitable for the patient may be found.

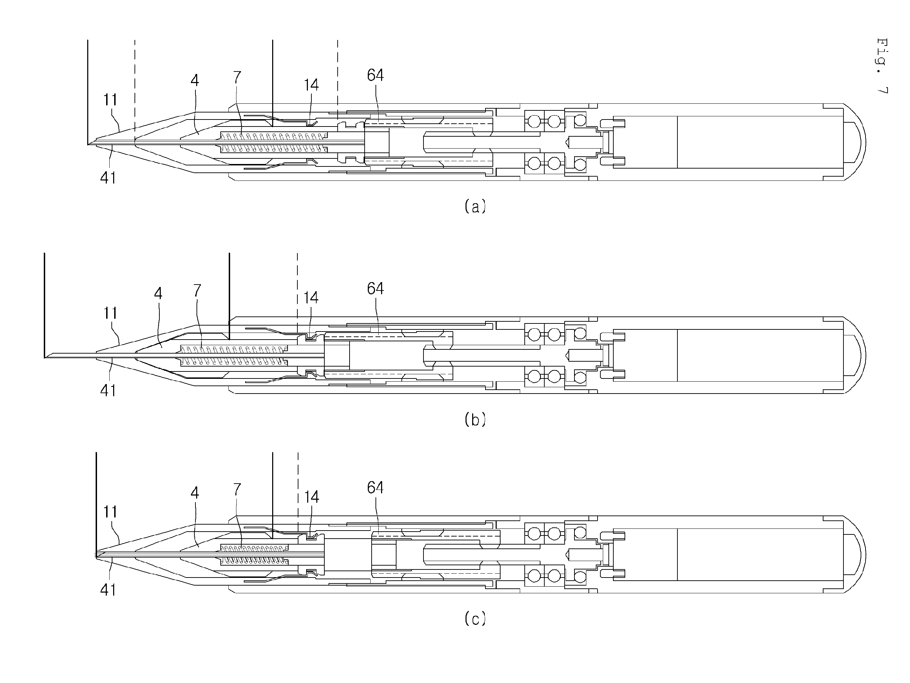

Fig. 6 only describes the motor 10 being put into operation to start the implanting apparatus. Fig. 7 is a time-series diagram to illustrate the mechanical operation of the implanting apparatus. Referring to Fig. 7, the operation is described in time series, focusing on the implanting needle portion 4 and the mandrel portion 5.

Fig. 7A illustrates a state in which the motor 10 remains idle before the operation. Here, medical personnel determines the direction in which the hair root is to inserted by using the concave portion 11, and places the hair root at the opening at the front of the implanting needle 41. Here, the implanting needle 41 is exposed to outside through the pin hole 12. The motor portion 6 and the implanting apparatus are put into operation when a signal is generated by the operation start signal generator 9 after medical personnel brings the front end of the front casing 1 close to the scalp of a patient.

Fig. 7B illustrates a state after the implanting apparatus is put into operation. Referring to Fig. 7B, the implanting needle portion 4 and the mandrel portion 5 are pushed forwards by the movable portion 64. The mandrel portion 5 does not move when the plate spring 14 is held by the groove 52 disposed at the mandrel portion 5. Here, the implanting needle 41 has penetrated the scalp at a certain depth while supporting the hair root. Also, the mandrel 51 has been pushed to the front end of the front casing 1, and therefore the hair root may remain in the scalp because of the mandrel 51 even when the implanting needle 41 retreats.

Then, the movable portion 64 begins to move backwards. In other words, the implanting needle 41 moves out of the scalp. However, the hair root must not be taken out of the scalp even when the implanting needle 41 is taken out. For the purpose, the mandrel 51 must maintain an original position. The operation may be achieved by the plate spring 14 remaining inserted into the groove 52 of the mandrel supporting portion 54. Since the implanting needle portion 4 is moved backwards and the mandrel portion 5 is not when the movable portion 64 is moved backwards, an elastic member 7 disposed in between is compressed. Also, the implanting needle 41 does not rotate because of an interaction between the groove portion 13 and the protruding portion 43 even when the implanting needle portion 4 is moved backwards. Therefore, the hair root does not rotate and hair implanting may be carried out in a direction desired by medical personnel.

With the movable portion 64 continuing to move backwards, a transforming portion 44 disposed at the implanting needle portion 4 may be further moved to the point of being able to extend the plate spring 14. When the plate spring 14 is extended, the plate spring 14 may be released from the groove 52. Fig. 7C illustrates a state immediately before the plate spring 14 is released from the mandrel supporting portion 54.

Referring to Fig. 7C, compression is continuing for the elastic member 7 disposed between the implanting needle portion 4 and the mandrel portion 5, but the transforming portion 44 provided to the implanting needle portion 4 is pushing and widening the plate spring 14. Therefore, the plate spring 14 is not going to hold the groove 52 and the mandrel portion 5 is going to be pushed backwards by the elastic member 7. By moving forwards a little bit again and leading implanting needle 41 to be exposed to outside through the pin hole 12, the motor portion 6 may make the implanting apparatus return to the state illustrated in Fig. 7A for repeated hair implanting.

The dotted lines in Figs. 7A, 7B, and 7C illustrate front ends of the mandrel and the mandrel supporting portion, and the solid lines illustrate front ends of the implanting needle and the transforming portion. The dotted and solid lines may help fully understand the movement of the implanting needle and the mandrel.

After one session of implantation is over, the mandrel portion and the implanting needle portion may be exchanged by separating the front casing from the middle casing.

Any reference in this specification to "one embodiment," "an embodiment," "example embodiment," etc., means that a particular feature, structure, or characteristic described in connection with the embodiment is included in at least one embodiment of the invention. The appearances of such phrases in various places in the specification are not necessarily all referring to the same embodiment. Further, when a particular feature, structure, or characteristic is described in connection with any embodiment, it is submitted that it is within the purview of one skilled in the art to effect such feature, structure, or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An implanting apparatus comprising:
a casing;
an implanting needle portion in the casing;
a mandrel portion forwardly and backwardly movable in relation to the implanting needle portion;
an elastic member disposed between the implanting needle portion and the mandrel portion;
a movable portion movable forwards and backwards to move the implanting needle portion;
a spring fixed relative to the casing so as to fix a position of the mandrel portion when the implanting needle portion is moved; and
a transforming portion movable together with the implanting needle portion for transforming the spring.

2. The implanting apparatus according to claim 1, wherein the casing is separable into multiple pieces, and a front casing of the multiple pieces comprises a concave portion in a front end thereof for guiding a direction of hair root insertion.

3. The implanting apparatus according to claim 1, further comprising a motor portion to move the movable portion forwards and backwards.

4. The implanting apparatus according to claim 1, wherein at least one of a rotary movement between the casing and the implanting needle portion, a rotary movement between the movable portion and the casing, and a rotary movement between the implanting needle portion and the mandrel portion is restricted.

5. An implanting apparatus comprising:
an operation start signal generator which generates an operation start signal when an outside signal is input;
a control unit which puts a motor into operation by receiving the signal generated by the operation start signal generator; and
the motor which moves the implanting needle portion forwards and backwards by following the control unit.

6. The implanting apparatus according to claim 5, further comprising an operation unit for controlling an operation status of the motor.
